# EUROPEAN PATENT APPLICATION

(11) **EP 4 523 746 A1**
(43) Date of publication of application: **19.03.2025**
(21) Application number: 23803380.7
(22) Date of filing: 20.04.2023
(51) Int. Cl.: A61P 3/02, A61P 31/12, A61P 37/04, A61P 43/00, C12R 1/225, C12N 1/20, A23L 33/135, A61K 35/747

(54) **NOVEL LACTIC ACID BACTERIUM, AND COMPOSITION, FOOD OR BEVERAGE, AND PHARMACEUTICAL PRODUCT CONTAINING LACTIC ACID BACTERIUM**

(30) Priority: 13.05.2022 JP 2022079346
(71) Applicant: MITSUBISHI GAS CHEMICAL COMPANY, INC., Chiyoda-ku Tokyo 100-8324 (JP)
(72) Inventor: SUGIMOTO, Atsushi, Tokyo 100-8324 (JP); NAKAMURA, Tomoe, Niigata-shi, Niigata 950-3112 (JP); CHIHAMA, Ryota, Niigata-shi, Niigata 950-3112 (JP); TAKENAKA, Yuto, Niigata-shi, Niigata 950-3112 (JP); SATO, Yuuki, Niigata-shi, Niigata 950-3112 (JP)
(74) Representative: Müller-Boré & Partner Patentanwälte PartG mbB
(86) International application number: PCT/JP2023/015823
(87) International publication number: WO 2023/218898

(57) **Abstract**

Provided are a lactic acid bacterium that can enhance IL-12p70 and IFN-λ production and thereby enhance the resistance to infection with infectious microorganisms and viruses, mitigate stress, and suppress excessive inflammatory response that causes symptom worsening; compositions, such as an immunostimulatory composition, a stress relief composition, and an inflammation suppression composition, containing the lactic acid bacterium; and food or beverage and pharmaceutical products containing these compositions. *Lactobacillus sakei* strain MG-LAB279 (accession number: NITE BP-03645); compositions, such as an immunostimulatory composition, a stress relief composition, and an inflammation suppression composition, containing the strain or a bacterial cell component thereof as an active ingredient; and food or beverage and pharmaceutical products containing the compositions.

## Description

### Technical Field

The present invention relates to *Lactobacillus sakei* strain MG-LAB279 (accession number: NITE BP-03645), which is a novel lactic acid bacterium, and to a composition, food or beverage, and a pharmaceutical product containing the lactic acid bacterium.

### Background Art

Interferon belongs to a cytokine family and has been identified as a factor that suppresses virus infection. It is now clear that interferon exhibits various activities, such as antitumor, anticancer, and cell proliferation suppression, in addition to immunoregulatory activities including antiviral activities. In humans, interferon is classified into type I interferon, type II interferon, and type III interferon. IFN-λ is a cytokine that was found in 2003 and is classified in type III interferon. As isoforms, IFN-λ1 (another name: IL-29), IFN-λ2 (another name: IL-28A), and IFN-λ3 (another name: IL-28B) are known. IFN-λ has been confirmed to be produced by dendritic cells, hepatic cells, intestinal epithelial cells, lung epithelial cells, and the like, and has an effect of activating natural immunity, which is a system to protect living bodies from bacteria, viruses, and the like, as in type I interferon.

IFN-λ induces expression of an IFN-inducible gene group (ISG), similar to in type I interferon, to suppress virus infection. Furthermore, IFN-λ is known to act cooperatively with type I interferon and thereby have an enhanced antiviral effect. Conventionally, IFN-λ has been thought to have a duplicated function with type I interferon. In recent years, it became clear that they act independently to exhibit the antiviral activity, which is attracting attention. The receptor of type I interferon is expressed in every cell and therefore induces systemic inflammation as part of the immune response, which may result in excessive inflammatory response. Since excessive inflammatory response due to virus infection causes severe symptoms such as multiple organ failure, it is important to suppress systemic excessive inflammatory response. In contrast, since the receptor of IFN-λ is present in only the above-mentioned hepatic cells, intestinal epithelial cells, lung epithelial cells, and the like, it also can be expected to impart selective bioactive effects with reduced side effects to the subject administered IFN-λ.

Immune responses in vivo are largely affected by the balance between inflammatory and anti-inflammatory substances. For example, interleukin-6 (IL-6) and tumor necrosis factor (TNFα), which are inflammatory cytokines, are involved in initial response to infectious microorganisms and amplify the inflammatory response. In contrast, anti-inflammatory cytokines such as IL-10 have an effect of suppressing inflammatory response. In general, although the immune response stops, for example, when the infection is resolved, overproduction of immunologic cytokines is caused by losing the regulatory function, which may cause systematic damages (immune system overdrive (cytokine storm)) in the host cell. As factors identified as causing immune system overdrive, for example, IL-6, TNFα, and interferon are known. In order to maintain the homeostasis of immune response, it is necessary to regulate the production of inflammatory cytokines and anti-inflammatory cytokines.

Interleukin-12p70 (IL-12p70) has effects such as differentiation induction of helper T precursor cells (Th0) to helper T cell type 1 (Th1) and activation of natural killer cells (NK cells). Th1 cells enhance the activity of phagocytic cells, such as T cells and monocytes, by production of interleukin-2 (IL-2), interferon-y (IFN-y), or the like and thereby enhance the resistance to infection of viruses, bacteria, and the like. NK cells have ability of specifically killing cancer cells, virus-infected cells, and the like. Accordingly, it can be expected to prevent cancer and the like by enhancing the resistance to virus infection through an enhance in the production of IL-12p70. Enhancement of resistance to virus infection means that the future symptoms of viral disease are improved, mitigated, or cured or that becoming conditions indicating being suffered from viral disease is prevented, suppressed, or delayed.

The resistance to virus infection is also affected by stress conditions. Stress is caused by responding to various stimuli (stressor) harmful to living bodies, and people in modern society are exposed to various types of stress such as changes in living and working environments, complex human relationships, and physical and mental fatigue. It is known that in a living body that has responded to stress, activities of the sympathetic-adrenomedullary system and the hypothalamic-pituitary-adrenocortical system are increased. A rise in the sympathetic-adrenomedullary system causes symptoms such as blood pressure increase, sweating, salivation, and awakening. It is known that a rise in the hypothalamic-pituitary-adrenocortical system enhances, for example, secretion of adrenocorticotropic hormone (ACTH) from the anterior pituitary gland to increase the blood levels of glucocorticoids such as cortisol, corticosterone, and cortisone. These glucocorticoids are generally called stress hormones. Many of the effects of glucocorticoids are involved in body response (activation of energy metabolism and promotion of cardiac function) to stress and play important roles in maintaining homeostasis. However, in contrast, it is known that glucocorticoids are harmful to living bodies such that proliferation and function of immune cells are decreased. Accordingly, in order to maintain the immune function, it is considered that it is important to relieve stress.

In order to improve the immune function for preventing virus infection, an immunostimulatory composition having immunostimulating activity is used. The immunostimulatory composition is desirably one that naturally occurs. In particular, food is desirable because it can be ingested on a daily basis with little risk of side effects. As food with immunostimulating activity, polysaccharides of Basidiomycetes represented by Shiitake mushrooms (Lentinus edodes) and agaricus, and edible microorganisms, such as lactic acid bacteria, Aspergillus oryzae, or yeast, and cell wall components thereof are known.

Patent Literature 1 describes that *Lactococcus lactis* subsp. *lactis* strain JCM5805 promotes IFN-λ production of dendritic cell pDC. Patent Literature 2 describes that *Tetragenococcus halophilus* strain KK221 promotes IFN-λ production of dendritic cell BDCA3DC. Patent Literature 3 describes IFN-λ production-promoting effect of bacteria of the genus *Bifidobacterium* as an active ingredient. Patent Literature 4 describes that *Lactobacillus paracasei* strain MCC1375 promotes IL-12 production and activates NK cells, providing a preventive effect on influenza virus infection.

### Citation List

### Patent Literature

Patent Literature 1
   Japanese Patent Laid-Open No. 2017-201984
Patent Literature 2
   International Publication No. WO 2017/175774
Patent Literature 3
   Japanese Patent Laid-Open No. 2019-167327
Patent Literature 4
   International Publication No. WO 2012/133827

### Summary of Invention

### Technical Problem

However, although agents and pharmaceutical products that regulate immune functions containing lactic acid bacteria including bacteria of the genus *Lactobacillus,* the genus *Lactococcus,* and the like as active ingredients have been disclosed, no agents containing lactic acid bacteria or compositions derived from lactic acid bacteria as main components and having effects of increasing IL-12p70 and IFN-λ production and sufficiently enhancing the resistance to virus infection have been disclosed, and no food or beverage having a strong preventive effect against virus infection has been developed.

It is an object of the present invention to provide an immunostimulatory composition, a stress relief composition, and an inflammation suppression composition (anti-inflammatory composition) that enhance IL-12p70 and IFN-λ production to activate natural immunity and are thereby effective for improving, mitigating, or treating symptoms of viral disease or preventing, suppressing, or delaying becoming conditions associated with viral disease.

### Solution to Problem

The present inventors intensively studied to solve the above problems and, as a result, found that *Lactobacillus sakei* strain MG-LAB279 enhances IL-12p70 and IFN-λ production and has excellent effects of preventing virus infection and the like, and the present invention has been accomplished.

In order to achieve the above object, the present invention provides the following lactic acid bacterium and a composition, food or beverage, and a pharmaceutical product including the lactic acid bacterium.

*[1] Lactobacillus sakei* strain MG-LAB279 (accession number: NITE BP-03645).
[2] A composition containing the strain according to [1] and/or a bacterial cell component thereof.
[3] An immunostimulatory composition containing the strain according to [1] and/or a bacterial cell component thereof as an active ingredient.
[4] The immunostimulatory composition according to [3], wherein the immunostimulatory composition does not induce inflammation.
[5] A virus infection preventing or treating composition containing the strain according to [1] and/or a bacterial cell component thereof as an active ingredient.
[6] An NK cell-activating composition containing the strain according to [1] and/or a bacterial cell component thereof as an active ingredient.
[7] An interferon λ production-promoting composition containing the strain according to [1] and/or a bacterial cell component thereof as an active ingredient.
[8] An interleukin-12p70 production-promoting composition containing the strain according to [1] and/or a bacterial cell component thereof as an active ingredient.
[9] A stress relief composition containing the strain according to [1] and/or a bacterial cell component thereof as an active ingredient.
[10] An inflammation suppression composition containing the strain according to [1] and/or a bacterial cell component thereof as an active ingredient.
[11] A skin-improving composition containing the strain according to [1] and/or a bacterial cell component thereof as an active ingredient.
[12] Food or beverage containing strain MG-LAB279 according to [1] or the composition according to any one of [2] to [11].
[13] A pharmaceutical product containing strain MG-LAB279 according to [1] or the composition according to any one of [2] to [11].

### Advantageous Effects of Invention

According to the present invention, it is possible to provide a lactic acid bacterium that can enhance IL-12p70 and IFN-λ production and thereby enhance the resistance to infection of infectious microorganisms and viruses, mitigate stress, and suppress excessive inflammatory response that causes symptom worsening; compositions, such as an immunostimulatory composition, a stress relief composition, and an inflammation suppression composition, containing the lactic acid bacterium; and food or beverage and pharmaceutical products containing these compositions.

### Brief Description of Drawings

[Figure 1] Figure 1 shows the measurement results (IFN-λ production levels) of Test Examples 2 and 3 in a graph.
[Figure 2A] Figure 2A shows the measurement and calculation results (MX1 gene expression level) of Test Example 2 in a graph.
[Figure 2B] Figure 2B shows the measurement and calculation results (OAS1 gene expression level) of Test Example 2 in a graph.
[Figure 3] Figure 3 shows the measurement results (result 1: serum corticosterone concentration) of Test Example 4 in a graph.
[Figure 4] Figure 4 shows the measurement and calculation results (result 2: IFN-λ gene expression level) of Test Example 4 in a graph.
[Figure 5] Figure 5 shows the measurement results (result 3: serum TNFα concentration) of Test Example 4 in a graph.
[Figure 6] Figure 6 shows the observation results (result 1: general condition score after influenza virus infection) of Test Example 5 in a graph.
[Figure 7] Figure 7 shows the observation results (result 2: survival rate after influenza virus infection) of Test Example 5 in a graph.
[Figure 8] Figure 8 shows the measurement results (result 3: virus count in the lung) of Test Example 6 in a graph.
[Figure 9] Figure 9 shows the measurement results (result 4: NK activity) of Test Example 6 in a graph.
[Figure 10] Figure 10 shows the observation results (result 1: general condition score after influenza virus infection) of Test Example 7 in a graph.
[Figure 11] Figure 11 shows the observation results (result 2: survival rate after influenza virus infection) of Test Example 7 in a graph.
[Figure 12] Figure 12 shows the measurement results (result 3: virus count in the lung) of Test Example 8 in a graph.
[Figure 13] Figure 13 shows the measurement results (result 4: NK activity) of Test Example 8 in a graph.
[Figure 14A] Figure 14A shows the observation results (result 5: pulmonary lesions in the bronchi) of Test Example 8 in a graph.
[Figure 14B] Figure 14B shows the observation results (result 5: pulmonary lesions in the alveoli) of Test Example 8 in a graph.
[Figure 15] Figure 15 shows the measurement results (IFN-λ production level) of Test Example 9 in a graph.
[Figure 16] Figure 16 shows the measurement results (IL-10 concentration) of Test Example 10 in a graph.
[Figure 17] Figure 17 shows the measurement results (result 1: body weight change rate after colitis induction) of Test Example 11 in a graph.
[Figure 18] Figure 18 shows the measurement results (result 2: length of large intestine after colitis induction) of Test Example 11 in a graph.
[Figure 19] Figure 19 shows the observation results (result 2: colitis symptom score after colitis induction) of Test Example 11 in a graph.

### Description of Embodiments

Embodiments of the present invention (hereinafter, referred to as "the embodiment") will now be described in detail, but the present invention is not limited thereto and can be variously modified without departing from the gist of the invention.

Strain MG-LAB279 (hereinafter, also simply referred to as "bacterial cell") belonging to *Lactobacillus sakei* of the embodiment is a lactic acid bacterium that has been deposited in the National Institute of Technology and Evaluation, Patent Microorganisms Depositary with accession number: NITE BP-03645 on May 2nd, 2022.

Strain MG-LAB279 is characterized by promoting interferon λ (IFN-λ) production and interleukin-12p70 (IL-12p70) production.

Strain MG-LAB279 can be provided as a composition containing the strain. Examples of the composition include, but are not limited to, an immunostimulatory composition (e.g., a virus infection-preventing or treating composition, an NK cell-activating composition, an interferon λ production-promoting composition, and an interleukin-12p70 production-promoting composition), a stress relief composition, an inflammation suppression composition, and a skin-improving composition. These compositions can be used as food or beverage, a pharmaceutical product, feed, or the like, which will be described in detail below.

The composition of the embodiment, such as an immunostimulatory composition, a stress relief composition, and an inflammation suppression composition, includes *Lactobacillus sakei* strain MG-LAB279 as an active ingredient. *Lactobacillus sakei* strain MG-LAB279 included in the composition of the embodiment may be a live bacterial cell or a dead bacterial cell or may be both, but dead bacterial cells are preferable.

The present inventors continued to select bacterial strains that also have excellent flavor and physical properties when applied to food from a large number of lactic acid bacteria derived from plants, fermented food, and humans, and were able to successfully select *Lactobacillus sakei* strain MG-LAB279. The present inventors studied and found that production of IL-12p70 and IFN-λ is enhanced by ingesting *Lactobacillus sakei* strain MG-LAB279. Consequently, it is expected that the natural immunity is activated by ingesting *Lactobacillus sakei* strain MG-LAB279 to enhance the resistance to disease caused by viruses and the like.

The composition of the embodiment, such as an immunostimulatory composition, a stress relief composition, and an inflammation suppression composition, uses a naturally occurring lactic acid bacterium, *Lactobacillus sakei* strain MG-LAB279, and therefore has an effect of extremely high safety.

As the culture medium for culturing *Lactobacillus sakei* strain MG-LAB279, various media, such as a milk medium, a medium containing milk components, and a semisynthetic medium not containing them, can be used. As such a medium, a reconstituted skim milk medium obtained by reconstituting skim milk and sterilizing it with heat can be exemplified.

*Lactobacillus sakei* strain MG-LAB279 is cultured by static culture or neutralized culture with constant pH control, but the culture method is not particularly limited as long as the conditions are good for bacterial growth. The bacterial cells are cultured according to a usual method for culturing lactic acid bacteria, and the substance isolated from the resulting culture by a collection method such as centrifugation can be directly used.

Dead bacterial cells can be usually obtained by heating bacterial cells. The heating conditions are not particularly limited as long as the bacterial cells die, but, in general, a sufficient result can be obtained by heating at 90°C for about 30 minutes.

*Lactobacillus sakei* strain MG-LAB279 that is contained in the composition of the embodiment, such as an immunostimulatory composition, a stress relief composition, and an inflammation suppression composition, is not limited to purely isolated bacterial cells and may be its culture, suspension, or another material containing the bacterial cells. In addition, cytosolic components such as nucleic acid or bacterial cell components such as a cell wall fragment, obtained by treating bacterial cells with an enzyme or physical means, may be used. These components may be used alone or in combination. Accordingly, dead bacterial cells of *Lactobacillus sakei* strain MG-LAB279 may be the culture or the bacterial cells themselves contained in the culture. In such a case, food or beverage, a pharmaceutical product, or the like including the composition of the embodiment, such as an immunostimulatory composition, a stress relief composition, or an inflammation suppression composition, may include a culture or bacterial cells themselves contained in the culture.

In the composition of the embodiment, such as an immunostimulatory composition, a stress relief composition, and an inflammation suppression composition, the bacterial cells may be mixed with dextrin for easier handling. In such a case, the composition of the embodiment, such as an immunostimulatory composition, a stress relief composition, and an inflammation suppression composition, can be used as a composition containing 0.1 to 99 mass% of dead bacterial cells of *Lactobacillus sakei* strain MG-LAB279 and 1 to 99.9 mass% of dextrin.

Live bacterial cells and/or dead bacterial cells of *Lactobacillus sakei* strain MG-LAB279 may be mixed in any food or beverage as a composition such as an immunostimulatory composition, a stress relief composition, or an inflammation suppression composition, or may be added to a raw material during the manufacturing process of food or beverage. Examples of the food or beverage include milk beverage, fermented milk, fruit juice beverage, soft beverage, jelly, candy, chocolate, dairy product, egg product such as mayonnaise, and confectionery and bread such as butter cake.

*Lactobacillus sakei* strain MG-LAB279 and/or its dead bacterial cells or culture may be mixed in any feed as a composition such as an immunostimulatory composition, a stress relief composition, or an inflammation suppression composition that is contained in livestock feed, or may be added to a raw material during the manufacturing process of feed.

Formulation of the composition of the embodiment, such as an immunostimulatory composition, a stress relief composition, and an inflammation suppression composition, is not particularly limited except that *Lactobacillus sakei* strain MG-LAB279 (such as dead bacterial cells) is used as an active ingredient, and formulation can be performed according to a usual method by appropriately mixing with an excipient, a stabilizer, a flavoring agent, and the like that are permitted in formulation.

The composition of the embodiment, such as an immunostimulatory composition, a stress relief composition, and an inflammation suppression composition, can also be formulated by mixing with an excipient, a binder, a disintegrant, a lubricant, a flavoring agent, a suspending agent, a coating agent, and other optional agents within a range not interfering with the effects of the bacterial cells or culture.

The composition of the embodiment, such as an immunostimulatory composition, a stress relief composition, and an inflammation suppression composition, can be used in a wide range of applications such as a pharmaceutical product, a quasi-drug, a cosmetic, and food and beverage. These compositions can be in a form of a tablet, a pill, a capsule, a granule, a powder, a powdered agent, a syrup, or the like, and are desirably orally administered in these forms.

In order to exhibit an immunostimulating activity, a stress relief activity, an inflammation suppression activity, and the like, the blending quantity may be adjusted such that, in an adult, 0.1 to 10 g, preferably 0.5 to 5 g, per day of dead bacterial cells of *Lactobacillus sakei* strain MG-LAB279 can be ingested. The content ratio of the lactic acid bacterium is not particularly limited and may be appropriately adjusted according to its use, ease of manufacturing, a preferable daily dosage, and the like.

The immunostimulatory composition of the embodiment can stimulate immunity without inducing inflammation and, in this point, overturns the common general technical knowledge that immunostimulation is accompanied by induction of inflammation, and has higher usefulness than conventional immunostimulatory compositions that are accompanied by induction of inflammation. The term "not induce inflammation" in the embodiment includes a case in which inflammation is not induced such that no side effect associated with inflammation is observed in a human or animal ingesting the immunostimulatory composition. "Not induce inflammation" can be verified by that, for example, in a test using an inflammation parameter represented by histopathological examination, as in Test Example 8 described later, an increase in the score indicating the inflammation degree is not observed (does not have a significant difference) compared to the control group in which virus infection treatment is performed.

### Examples

The present invention will now be described in more detail with reference to Examples and Test Examples, which are merely examples, and the present invention is not limited thereto in any way.

### [Identification of bacterial strain]

The bacterial strain can be identified by analyzing the 16S rRNA gene in accordance with the method described in "Science of Lactic Acid Bacteria and Bifidobacteria" published by Japan Society for Lactic Acid Bacteria (Kyoto University Press) (alternatively, analysis on contract may be adopted).

The 16S rRNA gene of strain MG-LAB279 was decoded by the above-mentioned known method. The decoded 16S rRNA gene of strain MG-LAB279 had a homology of 99.9% with *Lactobacillus sakei* subsp. *sakei* strain DSM20017, and it was thereby revealed that the bacterial strain belongs to *Lactobacillus sakei.*

### [Test Example 1]

### [Test for promoting production of IL-12p70 by mouse splenic cell]

Spleen cells collected from the spleen of a 6-week old male BALB/c mouse were adjusted to 5.0 × 10⁴ cells/well with a RPMI1640 (GIBCO) medium containing 10% fetal bovine serum (FBS, BIOWEST), 100 units/mL of antibiotics (penicillin-streptomycin) (GIBCO), and 50 µM mercaptoethanol (hereinafter, RPMI1640 medium), and were seeded in a 96-well plate (IWAKI). Twenty-one different strains of unknown lactic acid bacteria, including *Lactobacillus sakei* strain MG-LAB279, were added to the respective wells at 20 µg/mL and were cultured (in the control, no lactic acid bacterial strain was added). The culture was performed at 37°C under 5% CO₂.

### (ELISA assay)

The mouse-derived splenic cells were cultured for 48 hours, and 100 µL of the supernatant was collected from each well of the 96-well culture plate and was analyzed by ELISA. Washing in the ELISA assay was performed with a PBS buffer containing 0.05% Tween 20 using a plate washer (BIO-RAD, Immuno Wash 1575). To each well of a 96-well immunoplate (Thermo Fisher Scientific), 100 µL of a 0.8 µg/mL anti-mouse IL-12B monoclonal antibody (SIN Sino Biological Inc.) was added and left to stand at 4°C overnight for immobilization. After washing, 350 µL of 5% skim milk (FUJIFILM Wako Pure Chemical Corporation) was added thereto, followed by being left to stand at 4°C overnight for blocking. After washing, 50 µL of the culture supernatant was added thereto and was reacted at 37°C for 2 hours. After washing, 100 µL of a 0.8 µg/mL biotin-labeled anti-mouse IL-12 (p35) monoclonal antibody (MAB, Mabtech AB) was added thereto, followed by labelling at 37°C for 2 hours. After washing, 100 µL of a 0.8 µg/mL HRP-streptavidin conjugate (manufactured by Amersham plc) was added thereto, followed by reaction at room temperature for 1 hour. After washing, 100 µL of a TMB substrate (SeraCare) was added thereto for enzyme reaction, and 100 µL of 0.3 M sulfuric acid was then added thereto to stop the reaction. IL-12p70 was detected from the absorption at 450 nm with a plate reader (Perkin Elmer, ARVO X3). The production level of IL-12 was calculated from a standard curve produced using IL-12p70 standard samples (Pepro Tech). The measurement results are shown in Table 1 below (showing the results of only three lactic acid bacterial strains, which exhibited highest production levels of IL-12p70, including strain MG-LAB279, and also showing the result of Shield Lactic Acid Bacteria of Test Example 3). The two lactic acid bacteria other than strain MG-LAB279 shown in Table 1 are strain MG-LAB302 (accession number: NITE BP-03646) and strain MG-LAB533 (accession number: NITE BP-03647) that have been deposited in the National Institute of Technology and Evaluation, Patent Microorganisms Depositary on May 2nd, 2022.

**[Table 1]**

| **Lactic acid bacterium (strain)** | **Species** | **IL-12p70(pg/mL)** | **SD** |
|---|---|---|---|
| **Control** | | **90.5** | **21.9** |
| **MG-LAB279** | **Lactobacillus sakei** | **1758.5** | **148.5** |
| **MG-LAB302** | **Lactobacillus plantarum** | **604.6** | **159.1** |
| **MG-LAB533** | **Pediococcus pentosaceus** | **1252.3** | **280.3** |
| **Shield Lactic Acid Bacteria** | | **824.8** | **128.6** |

As a result, it was demonstrated that among the 21 lactic acid bacterial strains evaluated, *Lactobacillus sakei* strain MG-LAB279 most potently induces IL-12p70 production.

### [Test Example 2]

### [Test for promoting production of IFN-λ by human epithelial cell]

### (Preparation of lactic acid bacterium nucleic acid extract sample)

Each of cryopreserved lactic acid bacterial strains (21 strains that are the same as those in Test Example 1) was inoculated in 5 mL of an MRS medium (BD Difco) and was statically cultured at 30°C overnight. Cells were collected from these lactic acid bacterium culture solutions by centrifugation and were then resuspended in water, followed by centrifugation to obtain each bacterial cell pellet. Nucleic acid components were extracted from these bacterial cell pellets using a commercially available nucleic acid extraction kit (manufactured by Takara Bio Inc., NucleoBond AXG Column). Specifically, the bacterial cell pellet was suspended in a buffer, and at least one lytic enzyme of proteinase K, Lysozyme (FUJIFILM Wako Pure Chemical Corporation), or Labiase (OZEKI Co., Ltd.) was added thereto to perform treatment at 37°C overnight. A lytic buffer was added to these enzyme-treated solutions for treatment at 50°C for 30 minutes, followed by application to a column to obtain each nucleic acid eluate. The same quantity of isopropanol was added to each of these nucleic acid eluates, followed by inversion mixing and then centrifugation. The supernatant was discarded, and 70% ethanol was added to the resulting precipitate, followed by centrifugation. The supernatant was removed, and the resulting precipitate was dissolved in Nuclease Free Water (QIAGEN) to obtain a nucleic acid extraction sample of each lactic acid bacterial strain.

### (IFN-λ production test)

An HT-29 cell line obtained from the European Collection of Cell Cultures (ECACC) was cultured using 10 mL of a McCoy 5A (HyClone) medium containing 10% fetal bovine serum (FBS, BIOWEST) and penicillin-streptomycin 100 units/mL (Gibco) (hereinafter, McCoy medium) in a 10-cm culture dish. The culture was performed at 37°C under 5% CO₂. The HT-29 cells were cultured until reaching 70% to 80% confluence and were seeded in a 96-well tissue culture flat bottom plate (IWAKI) at a concentration of 4.0 × 10⁴ cells/well, followed by culturing overnight. The culture solution was removed, and 50 µL of the McCoy medium, 50 µL of the McCoy medium containing 1% Lipofectamin 2000 (Invitrogen), and 50 µL of the McCoy medium containing the nucleic acid extraction sample adjusted to a nucleic acid concentration of 15 µg/mL, 150 µL in total, were added to each well, followed by culturing for 20 hours. In a control not containing a sample, 150 µL of McCoy medium containing 0.33% Lipofectamin 2000 only was added to a well as a control. As a positive control, 50 µL of the McCoy medium containing 0.6 µg/mL Poly(:IC) (R&D Systems, Inc.) was added instead of the nucleic acid derived from a lactic acid bacterium to a well. The test was implemented by triplicate.

### (ELISA assay)

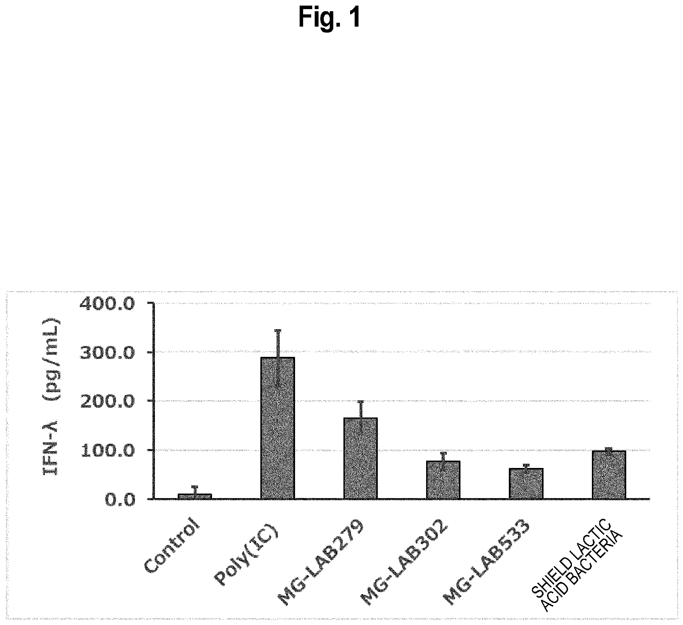

The HT-29 cells were cultured for 20 hours, and 100 µL of the supernatant was collected from each well of the 96-well culture plate. The production level of each sample was quantitatively measured using an ELISA assay kit (DuoSet ELISA Development System Human IL-29/IFN-λ1, R&D Systems Inc.). Specifically, each sample was prepared according to the attached protocol, and the absorbance of each sample was then measured at a wavelength of 450 nm. The production level of IFN-λ was quantitatively measured using a standard curve produced from the standard samples attached to the assay kit. The measurement results are shown in Figure 1 (showing the results of only top three lactic acid bacterial strains, which exhibited highest production levels of IFN-λ, and also showing the result of Shield Lactic Acid Bacteria of Test Example 3). The measurement values are shown by averages of triplicate, and error bars indicate standard errors.

As shown in Figure 1, it was demonstrated that among the 21 lactic acid bacterial strains evaluated, *Lactobacillus sakei* strain MG-LAB279 most potently induces IFN-λ production.

### [Verification of effect of preventing virus infection] (Analysis of expression level of MX1 and OAS1)

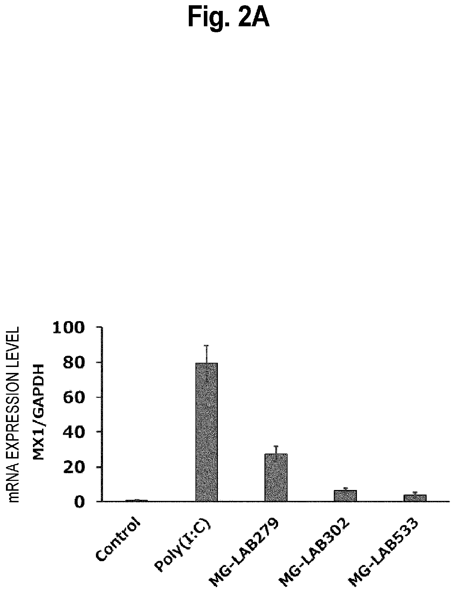

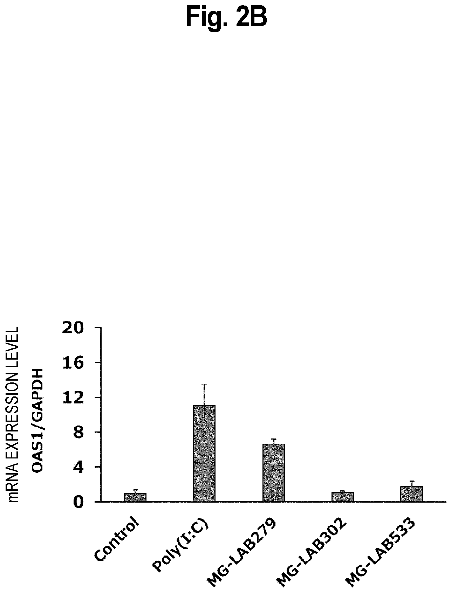

In the test, nucleic acid extraction samples prepared from *Lactobacillus sakei* strain MG-LAB279, *Pediococcus pentosaceus* strain MG-LAB533, and *Lactobacillus plantarum* strain MG-LAB302 were used. As a positive control, Poly(:IC) (R&D Systems Inc.) was used. The culture was performed by the same method as that in the IFN-λ production test. After the supernatant was removed, total RNA samples were prepared with CellAmp Direct RNA Prep Kit for RT-PCR (Takara Bio Inc.) and were used in analysis. In expression level analysis by quantitative PCR, One Step PrimeScript^{™} RT-PCR Kit (Takara Bio Inc.) and a quantitative PCR apparatus (Takara Bio Inc., TP-600) were used. MX1 was measured using primers of sequences 1 and 2 in Table 2, OAS1 was measured using primers of sequences 3 and 4, and GAPDH was measured using primers of sequences 5 and 6. Evaluation by expression level was performed by a relative value obtained by measuring the gene expression levels of MX1, OAS1, and a reference gene GAPDH and dividing the gene expression level by the reference gene expression level. The measurement results are shown in Figures 2A and 2B. The measurement values are shown by averages of quadruplicate, and error bars indicate standard errors.

**[Table 2]**

| SEQ ID NO | | Nucleotide sequence 5'→3' |
|---|---|---|
| 1 | Forward | ACA GGA CCA TCG GAA TCT TG |
| 2 | Reverse | CCC TTC TTC AGG TGG AAC AC |
| 3 | Forward | TGT CCA AGG TGG TAA AGG GTG |
| 4 | Reverse | CCG GCG ATT TAA CTG ATC CTG |
| 5 | Forward | GAA GGT GAA GGT CGG AGT C |
| 6 | Reverse | GAA GAT GGT GAT GGG ATT TC |

As shown in Figures 2A and 2B, it was demonstrated that *Lactobacillus sakei* strain MG-LAB279 potently promotes the expression of MX1 and OAS1. MX1 and OAS1 are known as proteins having interferon-inducing ability, and have effects of decomposing virus-derived RNA and inhibiting viral RNA replication. This suggests that *Lactobacillus sakei* strain MG-LAB279 has an effect of preventing virus infection.

### [Test Example 3]

### [Comparison with Shield Lactic Acid Bacteria (registered trademark)]

Shield Lactic Acid Bacteria (registered trademark) having a large share of the market was used as a Comparative Example and tested as in Test Examples 1 and 2. The measurement results are shown in Tables 1 and 3 and Figure 1.

**[Table 3]**

| | Control | MG-LAB279 | Shield Lactic Acid Bacteria (Comparative Example) |
|---|---|---|---|
| IFN-λ (pg/mL) | 9.1 | 165.6 | 97.2 |

It was shown in Tables 1 and 3 and Figure 1 that the lactic acid bacterium *Lactobacillus sakei* strain MG-LAB279 potently induces IL-12p70 and IFN-λ production and that the activity is higher than that of Shield Lactic Acid Bacteria (registered trademark) (*Lactobacillus paracasei* strain MCC1849) having a large share of the market. This demonstrates that dead bacterial cells of the lactic acid bacterium *Lactobacillus sakei* strain MG-LAB279 can be used as a cellular immune activator.

### [Test Example 4]

### [Analysis of IFN-λ expression level by mesenteric lymph node using immobilization-stressed mouse]

### (Breeding of mouse)

The test was performed using 8-week old female C57BL/6J mice (Charles River Laboratories Japan, Inc.) after preliminary breeding for one week. The animals were bred in a breeding room that was maintained at controlled temperature: 20°C to 26°C, controlled humidity: 40% to 70% RH, and light and darkness for 12 hours each (lighting: 7:00 a.m. to 7:00 p.m.). A sterilized polycarbonate flat-bottom cage (213 mm × 324 mm × 131 mm) containing animal bedding (Pulsoft, Oriental Yeast Co., Ltd.) was used from the period of the preliminary breeding. In order to consider the breeding environment, the animal bedding was replaced with new bedding twice a week.

### (Grouping of mice)

The mice were divided into 3 groups: a group fed with a normal diet (hereinafter, referred to as "control group"), a group fed with a normal diet and applied with an immobilization burden (hereinafter, referred to as "stress group"), and a group administered the lactic acid bacterium *Lactobacillus sakei* strain MG-LAB279 and applied with an immobilization burden (hereinafter, referred to as "strain 279 administration group"), respectively including 3, 5, and 4 mice.

### (Test food and administration method)

The test food in the control group and the stress group was powder feed (MF, Oriental Yeast Co., Ltd.). The test food in the strain administration group was the above powder feed including 0.5% of dead bacterial cells of the lactic acid bacterium *Lactobacillus sakei* strain MG-LAB279. After the grouping, the powder feed (MF) or the feed mixed with test substance was placed in a feeder (MF-4S, SHIN FACTORY) and was freely ingested. The feed was supplied or replaced with new feed twice a week. Administration of the test food was continued until the end of the test.

### (Method of stress treatment)

The 8-week old C57BL/6J mice in each group were bred for 12 days by free-feeding with the feed. The mice in the stress group and the strain 279 administration group were immobilized on the 13th day of breeding by being put in a 50-mL conical tube and using absorbent cotton and a silicon stopper so that the mice could not move. Considering the breeding environment, the conical tube for immobilization was provided with an air hole to prevent breath obstruction and an outlet for removing feces and urine. The immobilization was performed for 18 hours, and during the period, the mice were kept in water- and food-deprived conditions. The mice of the control group were bred as in other groups except that the immobilization was not performed.

### (Measurement of serum corticosterone)

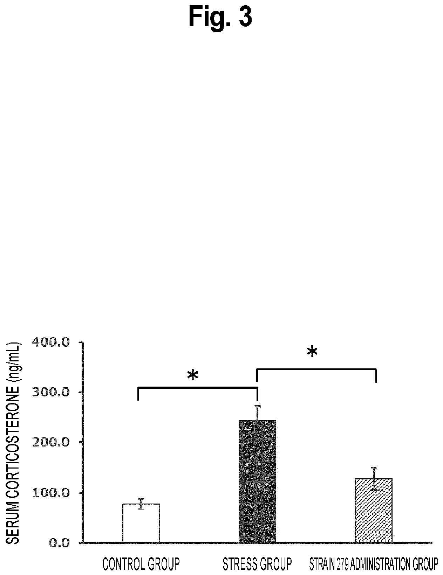

After the completion of the immobilization, dissection was performed under isoflurane anesthesia, and all blood was collected from the posterior vena cava. The obtained blood was dispensed in Capiject II (Terumo Co., Ltd.), and inversion mixing was performed 8 to 10 times. After leaving to stand at room temperature for 30 minutes, centrifugation at 3000 g for 10 minutes was performed to obtain serum. The concentration of corticosterone in the obtained serum was measured with Corticosterone ELISA Kit (Cayman Chemical Company). Verification was performed by two-group comparison between the stress group and the strain 279 administration group (unpaired t-test). The significance level was set at 5% ("*" in Figure 3).

### (Expression level analysis using mesenteric lymph node)

After the completion of the immobilization, autopsy was performed under isoflurane anesthesia, and the mesenteric lymph node was collected. The mesenteric lymph node was put in a 2-mL tube containing 1 mL of TRIzol^{™} Reagent (Invitrogen) and zirconia beads (Tomy Seiko Co., Ltd.) and was crushed with a bead-type cell crusher (Tomy Seiko Co., Ltd.), and 0.2 mL of chloroform was added to the resulting cell solution, followed by stirring. The aqueous layer was collected, and mesenteric lymph node-derived RNA was obtained by an isopropanol precipitation method. In expression level analysis by quantitative PCR, One Step PrimeScript^{™} RT-PCR Kit (Takara Bio Inc.) and a quantitative PCR apparatus (Takara Bio Inc., TP-600) were used. IFN-λ was measured using primers of sequences 7 and 8 in Table 4, and RPL19 was measured using primers of sequences 9 and 10. Evaluation by expression level was performed by a relative value obtained by measuring the gene expression levels of IFN-λ and a reference gene RPL19 and dividing the former by the latter.

**[Table 4]**

| SEQ ID NO | | Nucleotide sequence 5'→3' |
|---|---|---|
| 7 | Forward | ACC CTG AAG GTC TGG GAG AAA |
| 8 | Reverse | CTG TGG CCT GAA GCT GTG TA |
| 9 | Forward | ATG AGT ATG CTC AGG CTA CAG A |
| 10 | Reverse | GCA TTG GCG ATT TCA TTG GTC |

### (Measurement of inflammatory cytokine TNFα in serum)

After the completion of the immobilization, dissection was performed under isoflurane anesthesia, and all blood was collected from the posterior vena cava to prepare serum. The inflammatory cytokine TNFα in the serum was measured using Mouse TNFα ELISA Kit (Proteintech).

### (Result 1: Effect of test food on serum corticosterone concentration)

As shown in Figure 3, in the control group, the serum corticosterone concentration was increased by immobilization stress treatment compared to the untreated group. On the other hand, although an increase in the serum corticosterone concentration was observed also in the strain 279 administration group, the increase was significantly improved compared to the control group. This demonstrated that the lactic acid bacterium *Lactobacillus sakei* strain MG-LAB279 has an effect of relieving (reducing) stress caused by physical and mental fatigue and weakening the immune function.

### (Result 2: Effect of test food on IFN-λ expression level in mesenteric lymph node)

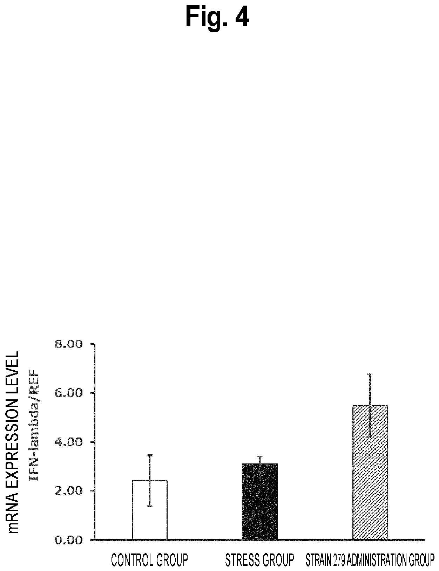

The measurement results are shown in Figure 4. It was confirmed that in the strain 279 ingestion group, the expression level of the IFN-λ gene in the mesenteric lymph node is high compared to the control group and the stress group. That is, it was demonstrated that even in a situation where the immune function may have been weakened by stress, expression of interferon λ in the mesenteric lymph node is accelerated by ingestion of bacterial cells of *Lactobacillus sakei* strain MG-LAB279.

### (Result 3: Effect of test food on serum TNFα)

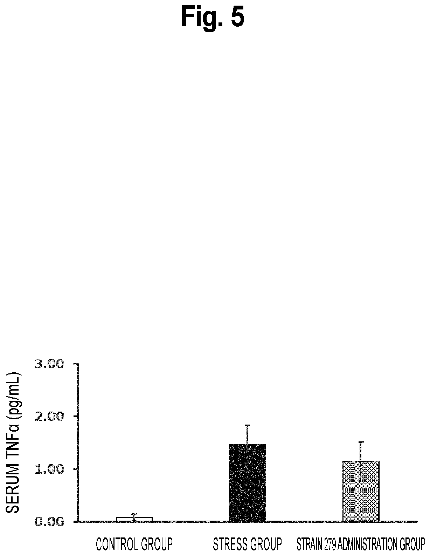

The measurement results of inflammatory cytokine TNFα in serum are shown in Figure 5.

As shown in Figure 5, in the control group, the serum TNFα concentration was increased by immobilization stress treatment compared to the untreated group. On the other hand, although an increase in the serum corticosterone concentration was observed also in the strain 279 administration group, the increase was suppressed compared to the control group. This demonstrated that the lactic acid bacterium *Lactobacillus sakei* strain MG-LAB279 can be expected to suppress the systemic inflammation accompanied with stress, in addition to an effect of relieving (reducing) stress weakening the immune function. It was also demonstrated that since it is possible to relieve stress and suppress inflammation, effects of improving skin problems, such as dry or chapped skin and rough skin, caused by stress or inflammation can be expected.

### [Test Example 5]

### [Evaluation of condition score and survival rate of influenza-infected mouse (part 1)]

### (Breeding of mouse)

The test was performed using 5-week old female BALB/c mice (BALB/c Cr Slc) (Japan SLC, Inc.) after preliminary breeding for one week. The animals were bred in a breeding room that was maintained at controlled temperature: 18°C to 28°C, controlled humidity: 30% to 80% RH, and light and darkness for 12 hours each (lighting: 6:00 a.m. to 6:00 p.m.). A sterilized plastic cage (W: 175 mm × D : 245 mm × H: 125 mm) containing animal bedding was used from the period of the preliminary breeding. In order to consider the breeding environment, environmental enrichment (nesting sheet, Animec) was placed in each cage.

### (Grouping of mice)

The mice were divided into 5 groups: an influenza virus infection-untreated group (hereinafter, referred to as "untreated group"), a control group treated with influenza virus infection (hereinafter, referred to as "control group"), a group administered with the lactic acid bacterium *Lactobacillus sakei* strain MG-LAB279 before influenza virus infection treatment (hereinafter, referred to as "strain 279 administration group"), a group administered with *Pediococcus pentosaceus* strain MG-LAB533 before influenza virus infection treatment (hereinafter, referred to as "strain 533 administration group"), and a group administered with *Lactobacillus plantarum* strain MG-LAB302 before influenza virus infection treatment (hereinafter, referred to as "strain 302 administration group"), each including 8 mice.

### (Test food and administration method)

The test food in the untreated group and the control group was powder feed (MF, Oriental Yeast Co., Ltd.). The test food in the strain 279 administration group was powder feed mixed with 0.5% of dead bacterial cells of the lactic acid bacterium *Lactobacillus sakei* strain MG-LAB279. The test food in the strain 533 administration group was powder feed mixed with 0.5% of dead bacterial cells of the lactic acid bacterium *Pediococcus pentosaceus* strain MG-LAB533. The test food in the strain 302 administration group was powder feed mixed with 0.5% of dead bacterial cells of the lactic acid bacterium *Lactobacillus plantarum* strain MG-LAB302. After the grouping, the powder feed (MF) or the feed mixed with a test substance was placed in a feeder and was freely ingested. The feed was replaced with new feed twice a week, and uneaten feed was discarded. Administration of the test food was continued until the end of the test.

### (Method of infection with influenza virus)

In infection treatment of mice with influenza virus, influenza virus PR8 [A/PR/8/34 (H1N1)] was used. A cryopreserved inoculation virus stock solution was thawed at time of use and prepared to 2 × 10³ PFU/mL, which was used as an inoculation virus solution. On the virus infection day (15th day after starting the test), the inoculation virus solution was instilled into the nasal cavity of each mouse at 0.05 mL (1 × 10² PFU/mouse) using a micropipette. The inoculation virus solution was stirred for each inoculation.

### (Measurement of body weight)

The body weight was measured with an electronic balance once a week during from the starting day of ingestion of the test food to the day before virus infection (14th day after starting the test) and every day after the day of the day of virus infection (15th day after starting the test). When 20% or more of decrease in the body weight based on the body weight measured on the day of virus infection was observed, euthanasia was performed by exsanguination from the abdominal aorta under isoflurane anesthesia. The euthanized animal was excluded from the measurement object.

### (Measurement of general condition score)

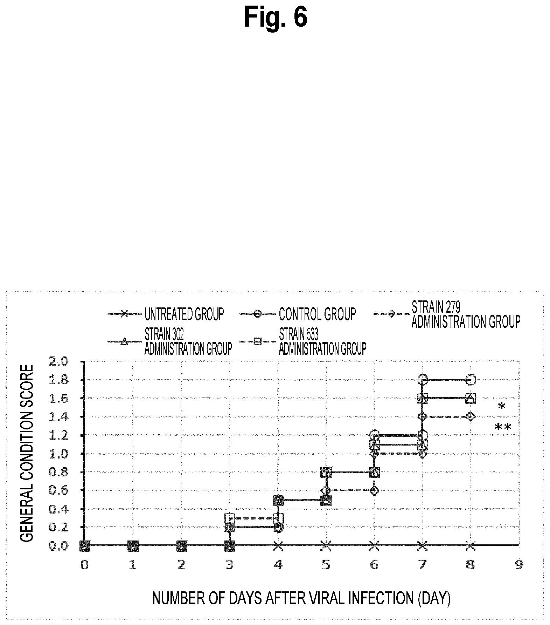

Observation of general conditions was implemented once a day after the day of virus infection. The general condition score was calculated as the total score for each affected area using the general condition score sheet of Table 5. When two or more affected areas of score 3 in the general condition score were observed, euthanasia was performed by exsanguination from the abdominal aorta under isoflurane anesthesia. The euthanized animal was excluded from the measurement object. Figure 6 shows general condition scores over time up to 7 days after influenza virus infection, using the day of infection as day 0. The values are each represented by mean ± standard error, and, in comparison with the control group, when the P value in the Wilcoxon rank sum test is less than 0.05, it was determined to have a statistically significant difference and marked with "*", and when the P value is less than 0.01, it was marked with "**".

**[Table 5]**

| Score | Affected area | | | |
|---|---|---|---|---|
| | (1) Eve | (2) Hair | (3) Behavior | (4) Other |
| 3 | Blepharosynechia (eyelids unable to open) | Very bad hair | Decrease of spontaneous movement (not move even when touched) | Respiratory failure, emaciation, prone position, and hypothermia |
| 2 | Disappearance of eyelid reflex (eyes open when touched, but there is no blink reflex) | Poor hair gloss and erect hair | Decrease of spontaneous movement (start to move when touched) | Breathing irregularities (tachypnea and bradypnea), emaciation |
| 1 | Eyelid closure (eyes open when touched) | Slightly erect hair | Showing interest in the environment, and responding to stimuli | Breathing irregularities (tachypnea or bradypnea) |
| 0 | Normal (there is a sparkle in the eyes) | Normal (the hair is good) | Normal | Normal (No irregularities in breathing and the like) |

### (Measurement of survival rate)

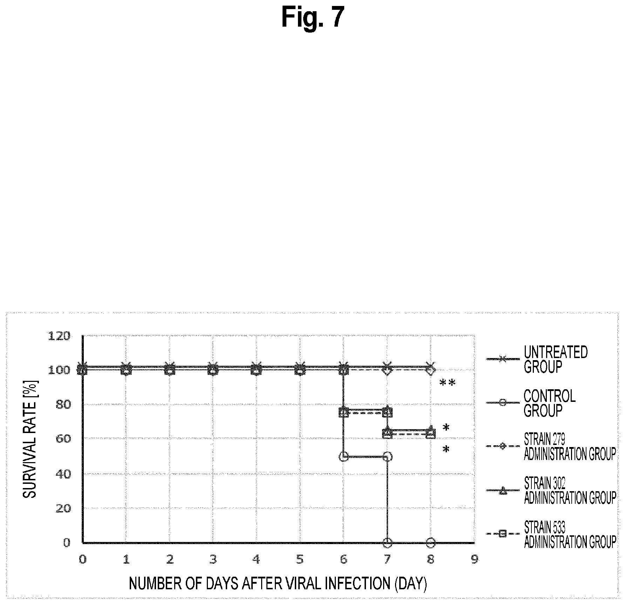

When a body weight decrease of 20% or more or two or more affected areas of score 3 in the general condition score was observed, on humanitarian grounds, euthanasia was performed by exsanguination from the abdominal aorta under isoflurane anesthesia. The euthanized animal was counted as dead on the day when calculating the survival rate. Figure 7 shows survival rates over time up to 7 days after influenza virus infection, using the day of infection as day 0. The values are each represented by mean ± standard error, and, in comparison with the control group, when the P value in the Logrank test is less than 0.05, it was determined to have a statistically significant difference and marked with "*", and when the P value is less than 0.01, it was marked with "**".

### (Result 1: Effect of test food on general condition score)

As shown in Figure 6, in the control group, the general condition score increased from the 3rd day after influenza virus infection. On the other hand, in the strain 279 administration group, although an increase in the general condition score by the influenza virus infection was observed, the condition improved compared to the control group. In also the strain 302 administration group and the strain 533 administration group, the condition improved compared to the control group, but the improvements were not as significant as in the strain 279 administration group.

### (Result 2: Effect of test food on survival rate)

As shown in Figure 7, in the control group, dead individuals were observed from the 6th day after influenza virus infection, and all individuals died on the 8th day after infection. In contrast, in the strain 279 administration group, no dead individuals were observed. In the strain 533 administration group and the strain 302 administration group, although an increase in the survival rate was observed compared to the control group, the improvements were not as significant as in the strain 279 administration group.

### [Test Example 6]

### [Virus count in the lung and evaluation of NK activity of influenza-infected mouse (part 1)]

Breeding of mice, test food, the administration method of test food, and the method of infection with influenza virus were performed as in Test Example 5. Grouping of mice was performed as in Test Example 5 except that each group used five mice. Euthanasia was performed by exsanguination from the abdominal aorta under isoflurane anesthesia on the 5th day (20th day after starting the test) from the day of virus infection (15th day after starting the test), and measurement of virus count in the lung and evaluation of NK activity were performed.

### (Measurement of virus count in the lung)

The lung was excised from a mouse and cut into fine pieces and was then homogenized in 2 mL of Hank's balanced salt solution (HBSS, Life Technologies Corporation) with a stirrer (Hiscotron (registered trademark), NS-51, Microtech Co., Ltd.). The extract was filtered through sterilized mesh (100 µm, cell strainer: FalconTM) to remove cell aggregation and impurities, and centrifugation (190 × g, for 5 minutes) with a centrifuge (AX-310, Tomy Seiko Co., Ltd.) was performed. The supernatant was used as a tissue solution and dispensed in an amount of about 1 mL into each of serum tubes and cryopreserved.

The lung tissue solution was thawed and appropriately diluted with minimum essential medium eagle (MEM, SIGMA) to produce a diluted solution. MDCK cells were seeded to a 12-well plate, and the undiluted and diluted lung tissue solutions were added in an amount of 0.1 mL to each well to allow the virus to adsorb to the cells for 1 hour.

Medium A (10 × MEM: 10 mL, 7.5% sodium hydrogen carbonate: 3 mL, 200 mmol/L L-glutamine: 2 mL, 1% DEAE dextran: 1 mL, 15% glucose: 1 mL, 10% BSA: 1 mL, 2.5% trypsin: 40 µL, antibacterial agent: 1 mL, sterile distilled water: 31 mL) that was used in the measurement of the concentration of the virus stock solution and medium B (agarose: 0.8 g, distilled water: 50 mL) were mixed in equal amounts, and 1.5 mL thereof was overlaid on the cells in each well. After the agarose solution solidified, culture was performed with a carbon dioxide culture apparatus for 2 days. Subsequently, a medium prepared by adding neutral red to a medium mixture of medium A and medium B was overlaid, and the virus plaques were counted on the following day. The virus count was calculated by using the number of plaques calculated from the maximum dilution ratio at which the number of plaques could be measured. If all samples were unmeasurable, samples were prepared again.

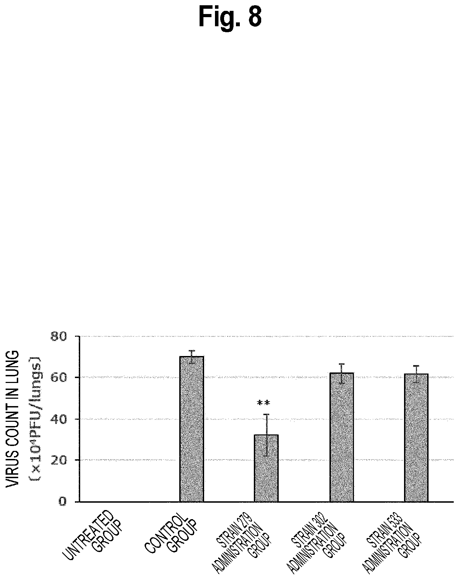

Figure 8 shows virus counts (PFU/Lung) in lung tissue on the 5th day after influenza virus infection treatment (20th day after starting the test). The values are each represented by mean ± standard error, and, in comparison with the control group, when the P value in the Wilcoxon rank sum test is less than 0.01, it was determined to have a statistically significant difference and marked with "**".

### (Evaluation of NK activity)

HBSS was added to the spleen excised from a mouse, and the spleen was cut into fine pieces and was gently crushed with the rubber part of the inner cylinder of a plastic syringe in a plastic dish to produce 2 mL of a single cell suspension. The suspension was sieved through a cell strainer to remove tissue residue, and the cell suspension was then put in a 50 mL centrifuge tube and centrifuged (1000 rpm, 190 × g, 5 minutes). The supernatant was removed, and 2 mL of a red blood cell lysis solution (water for injection: The Pharmacopoeia of Japan, Otsuka Distilled Water, Otsuka Pharmaceutical Factory, Inc.) was then added thereto. After thoroughly mixing for 3 seconds, an equal amount of 2 × PBS was added thereto. The supernatant was collected in such a manner that the precipitate is not collected and was centrifuged (1000 rpm, 190 × g, 5 minutes). The cells were washed with a culture solution and then resuspended in 1 mL of RPMI 1640 containing 10% FBS. Live cells were counted with trypan blue solution, and the cell count was adjusted to an appropriate number (effector cells).

A cell suspension of YAC-1 cells was transferred to a 15-mL centrifuge tube, and centrifugation (1000 rpm, 190 × g, for 5 minutes) was performed. The supernatant was removed, and 1 mL of RPMI 1640 was added thereto. The mixture was thoroughly mixed, and the cell count was adjusted to an appropriate number using a hemocytometer (target cells).

The cytotoxic ratio measured using the prepared effector cells and target cells with LDH Cytotoxicity Detection Kit (Takara Bio Inc.) was evaluated as NK activity.

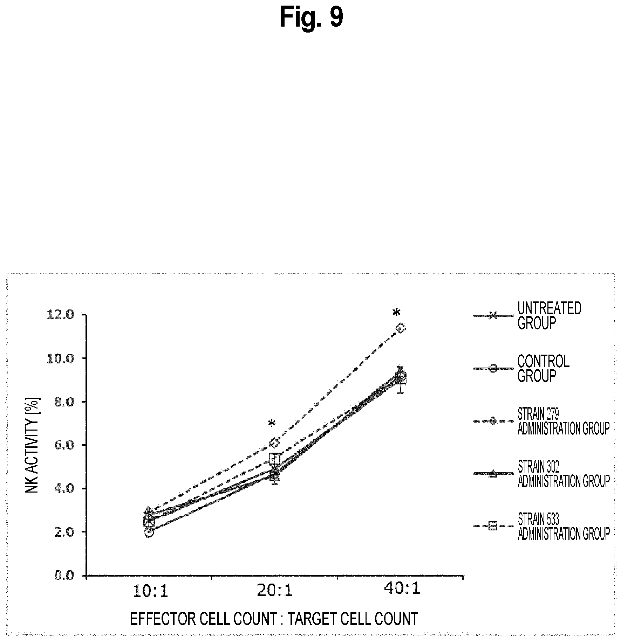

Figure 9 shows the NK activity in mouse spleen cells on the 5th day after influenza virus infection treatment (20th day after starting the test). The horizontal axis indicates the ratio of the number of effector cells and the number of target cells. That is, the cytotoxic ratios were measured by mixing mouse spleen cells and YAC-1 cells at 10 : 1, 20 : 1, and 40 : 1, and the measurement results were shown as NK activities. The values are each represented by mean ± standard error, and, in comparison with the control group, when the P value in the Wilcoxon rank sum test is less than 0.05, it was determined to have a statistically significant difference and marked with "*".

### (Result 3: Effect of test food on virus count in the lung)

As shown in Figure 8, no virus was detected in the untreated group, but the virus count in the control group increased. In contrast, although an increase in the virus count in the lung by influenza virus infection was observed also in the strain 279 administration group, the increase was less than that in the control group. In the strain 302 administration group and the strain 533 administration group, no reduction in the virus count in the lung was observed. This demonstrated that the survival rate in the strain 279 administration group is improved due to the antiviral effect of the strain 279 in reducing the virus count in vivo.

### (Result 4: Effect of test food on NK activity)

As shown in Figure 9, the NK activity in the spleen cells was increased in the strain 279 administration group compared to the control group. In contrast, in the strain 533 administration group and the strain 302 administration group, no increase in the NK activity was observed relative to the control group. This demonstrated that strain 279 has an effect of activating NK cells.

### [Test Example 7]

### [Evaluation of condition score and survival rate of influenza-infected mouse (part 2)]

### (Breeding of mouse)

The mice were bred using the same method as in Test Example 5.

### (Grouping of mice)

The mice were divided into 5 groups: a group not treated with influenza virus infection (hereinafter, referred to as "untreated group"); a control group treated with influenza virus infection (hereinafter, referred to as "control group"); a group administered with lactic acid bacterium *Lactobacillus sakei* strain MG-LAB279 before influenza virus infection treatment (hereinafter, referred to as "strain 279 administration group"); a group administered with *Lactococcus lactis* subsp. *lactis* strain JCM5805 before influenza virus infection treatment (hereinafter, referred to as "strain JCM5805 administration group"); and a group administered with Shield Lactic Acid Bacteria before influenza virus infection treatment (hereinafter, referred to as "Shield Lactic Acid Bacteria administration group"), each consisting of 8 mice.

### (Test food and administration method)

The test food in the untreated group and the control group was powder feed (MF, Oriental Yeast Co., Ltd.). The test food in the strain 279 administration group was the powder feed mixed with 0.5% of dead bacterial cells of the lactic acid bacterium *Lactobacillus sakei* strain MG-LAB279. The test food of the strain JCM5805 administration group was the powder feed mixed with 0.5% of dead bacterial cells of lactic acid bacterium *Lactococcus lactis* subsp. *lactis* strain JCM5805. The test food of the Shield Lactic Acid Bacteria administration group was the powder food mixed with 3.3% of Shield Lactic Acid Bacteria (such that the dead bacterial cells of the lactic acid bacterium was 0.5%). The administration method was the same as that in Test Example 5.

### (Method of infection with influenza virus)

Mice were infected with influenza virus as in Test Example 5.

### (Measurement of body weight)

The body weight was measured as in Test Example 5.

### (Measurement of general condition score)

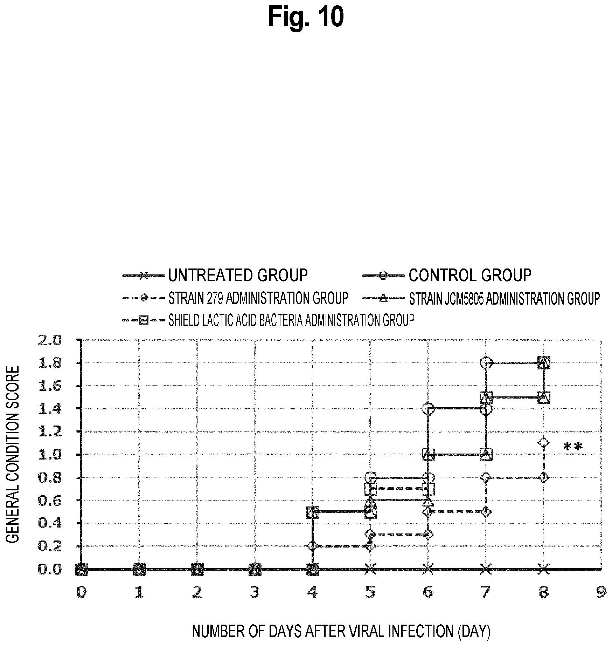

The general condition score was measured as in Test Example 5. The general condition score was calculated as the total score for each affected area using the general condition score sheet of Table 5. When two or more affected areas of score 3 in the general condition score were observed, euthanasia was performed by exsanguination from the abdominal aorta under isoflurane anesthesia. The euthanized animal was excluded from the measurement object. Figure 10 shows general condition scores over time up to 8 days after influenza virus infection, using the day of the infection as day 0. The values are each represented by mean ± standard error, and, in comparison with the control group, when the P value in the Wilcoxon rank sum test is less than 0.05, it was determined to have a statistically significant difference and marked with "*", and when the P value is less than 0.01, it was marked with "**".

### (Measurement of survival rate)

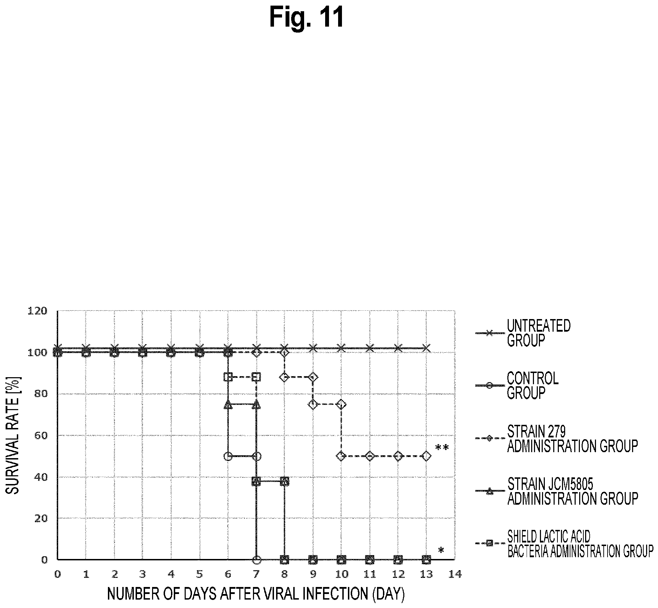

The survival rate was measured as in Test Example 5. Figure 11 shows survival rates over time up to 13 days after influenza virus infection, using the day of the infection as day 0. The values are each represented by mean ± standard error, and, in comparison with the control group, when the P value in the Logrank test is less than 0.05, it was determined to have a statistically significant difference and marked with "*", and when the P value is less than 0.01, it was marked with "**".

### (Result 1: Effect of test food on general condition score)

As shown in Figure 10, in the control group, the general condition score increased from the 4th day after influenza virus infection. In contrast, although an increase in the general condition score by influenza virus infection was observed also in the strain 279 administration group, the condition improved compared to the control group. Also in the strain JCM5805 administration group and the Shield Lactic Acid Bacteria administration group, the condition improved compared to the control group, but the improvements were not as significant as in the strain 279 administration group.

### (Result 2: Effect of test food on survival rate)

As shown in Figure 11, in the control group, dead individuals were observed from the 6th day after influenza virus infection, and all individuals died on the 7th day after infection. Also in the strain JCM5805 administration group and the Shield Lactic Acid Bacteria administration group, dead individuals were observed from the 6th day after infection, and all individuals died on the 8th day after infection. In contrast, in the strain 279 administration group, although dead individuals were observed on the 8th day after infection, half of the individuals survived even on the 13th day after infection. In the strain 279 administration group, a significant increase in the survival rate was observed compared to the control group. In the Shield Lactic Acid Bacteria administration group, although a significant increase in the survival rate was observed compared to the control group, the improvements were not as significant as in the strain 279 administration group.

### [Test Example 8]

### [Virus count in the lung and evaluation of NK activity of influenza-infected mouse (part 2)]

Breeding of mice, test food, the administration method of test food, and the method of infection with influenza virus were performed as in Test Examples 5 and 6. Grouping of mice was performed as in Test Example 7 except that each group used five mice. Euthanasia was performed by exsanguination from the abdominal aorta under isoflurane anesthesia on the 5th day (20th day after starting the test) from the day of virus infection (15th day after starting the test), and measurement of virus count in the lung and evaluation of NK activity were performed.

### (Measurement of virus count in the lung)

In measurement of virus count in the lung, the right lungs collected from mice were used. The measurement of virus count was performed as in Test Example 6.

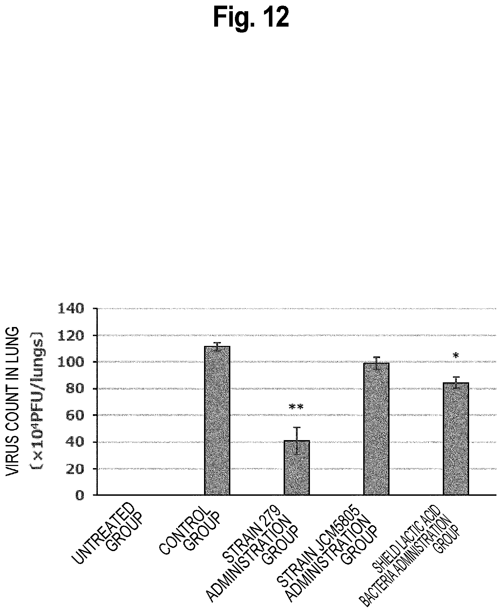

Figure 12 shows the virus counts (PFU/Lung) in lung tissue on 5th day after influenza virus infection treatment (20th day after starting the test). The values are each represented by mean ± standard error, and, in comparison with the control group, when the P value in the Wilcoxon rank sum test is less than 0.01, it was determined to have a statistically significant difference and marked with "**".

### (Evaluation of NK activity)

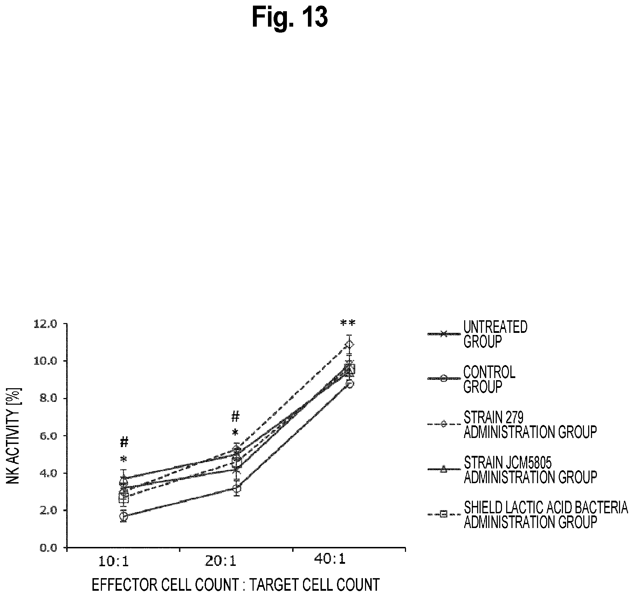

The NK activity was evaluated as in Test Example 6. Figure 13 shows NK activities in mouse spleen cells on the 5th day after influenza virus infection treatment (20th day after starting the test). The horizontal axis indicates the ratio of the number of effector cells and the number of target cells. That is, the cytotoxic ratios were measured by mixing mouse spleen cells and YAC-1 cells at 10 : 1, 20 : 1, and 40 : 1, and the measurement results were shown as NK activities. The values are each represented by mean ± standard error, and, in comparison with the control group, when the P value in the Wilcoxon rank sum test is less than 0.05, it was determined to have a statistically significant difference and marked with "* (strain 279 administration group)" and "# (strain JCM5805 administration group)".

### (Histopathological examination of the lung)

In histopathological examination, the left lungs collected from mice were used. The left lungs were fixed, and paraffin sections were prepared for all mice in each group according to a usual method, and the paraffin sections were stained with HE. Optical microscopic examination was implemented, and the paraffin section of the representative example of each group was photographed with a digital camera. Histopathological findings were scored in 5 levels based on the staining rate in the entire paraffin section, i.e., 0: no change (-, 0% of the total), 1: significantly light (±, 0% < to ≤ 25%), 2: light (+, 25% < to ≤ 50%), 3: moderate (++, 50% < to ≤ 75%), and 4: severe(+++, 75% < to ≤ 100%).

In the bronchi, total score of three items: hyperplasia and hypertrophy of the mucosal epithelium; degeneration and necrosis of the mucosal epithelium; and infiltration of mononuclear and polymorphonuclear leukocytes, was calculated. In the alveoli, total score of five items: atelectasis, edema, hemorrhage, infiltration of mononuclear and polymorphonuclear leukocytes (alveolar septum), and exudation of mononuclear and polymorphonuclear leukocytes, was calculated.

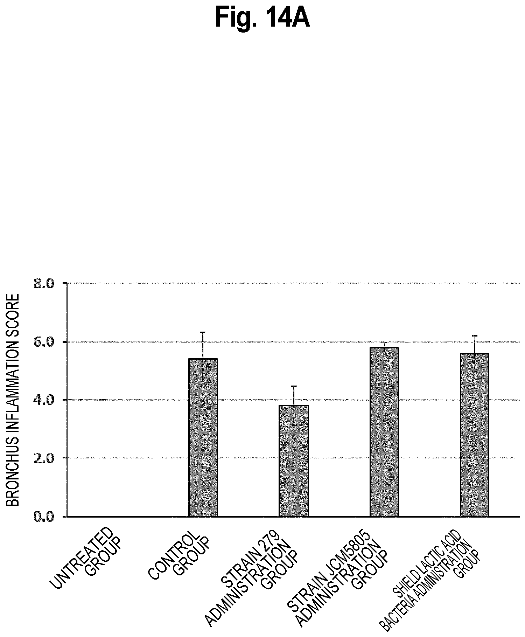

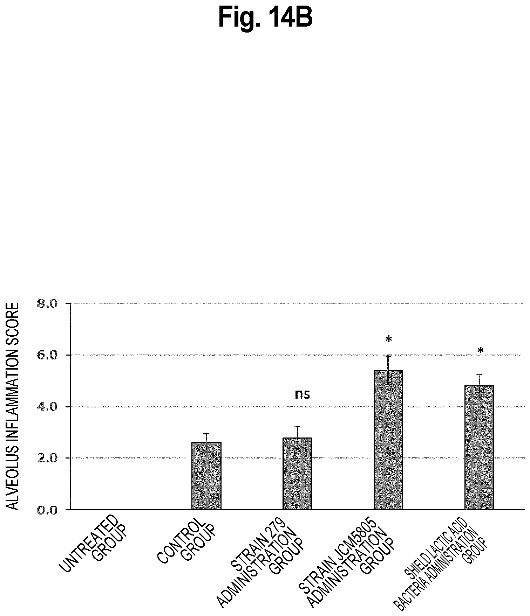

Figure 14 shows the scores calculated in histopathological examination of the left lungs on the 5th day after influenza virus infection treatment (20th day after starting the test). The values are each represented by mean ± standard error, and, in comparison with the control group, when the P value in the Wilcoxon rank sum test is less than 0.05, it was determined to have a statistically significant difference and marked with "*".

### (Result 3: Effect of test food on virus count in the lung)

As shown in Figure 12, in contrast to the untreated group where no virus was detected, the virus count increased in the control group. On the other hand, in the strain 279 administration group, an increase in the virus count in the lung by influenza virus infection was observed, but it was low compared to the control group. Also in the Shield Lactic Acid Bacteria administration group, the virus count was lower than that in the control group, but the degree thereof was not as much improvement as the strain 279 administration group. In the strain JCM5805 administration group, the virus count in the lung was not low compared to the control group. This demonstrated that the survival rate in the strain 279 administration group is improved due to the antiviral effect of the strain 279 in reducing the virus count in vivo.

### (Result 4: Effect of test food on NK activity)

As shown in Figure 13, in the strain 279 administration group, regardless of the cell ratio between the mouse spleen cells and the YAC-1 cells, the NK activity in the spleen cells was increased compared to the control group. In the strain JCM5805 administration group, when the cell ratio between the mouse spleen cells and the YAC-1 cells was 10 : 1 and 20 : 1, the NK activity in the spleen cells increased. On the other hand, in the Shield Lactic Acid Bacteria administration group, no increase in the NK activity was observed compared to the control group. This demonstrated that strain 279 and strain JCM5805 have an effect of activating NK cells.

### (Result 5: Effect of test food on pulmonary lesion)

As shown in Figures 14A and 14B, in contrast to the untreated group where no pulmonary lesions were observed in both the bronchus and alveoli, in the control group, the scores of both the bronchus and the alveoli increased and pulmonary lesions occurred. Also in the strain 279 administration group, an increase in the score by influenza virus infection was observed, but no significant change compared to the control group was observed. On the other hand, in the strain JCM5805 administration group and the Shield Lactic Acid Bacteria administration group, compared to the control group, a significant increase in the score in the alveoli was recognized, although a significant difference in the score in the bronchus was not observed. This demonstrated that, in contrast to strain JCM5805 and Shield Lactic Acid Bacteria, which activate immunity and simultaneously worsen the lung lesion by the influenza virus infection, strain 279 activates immunity but does not induce inflammation in the bronchus and alveoli.

### [Test Example 9]

### [Test for promoting production of IFN-λ by mouse splenic cell]

Spleen cells collected from the spleen of a 9-week old female C57BL/6J mouse were adjusted to 3.0 × 10⁴ cells/well with an RPMI 1640 (GIBCO) medium containing 10% fetal bovine serum (FBS, BIOWEST), 100 units/mL of antibiotics (penicillin-streptomycin) (GIBCO), and 50 µM mercaptoethanol (hereinafter, RPMI 1640 medium), and were seeded in a 96-well plate (IWAKI). *Lactobacillus sakei* strain MG-LAB279, *Pediococcus pentosaceus* strain MG-LAB533, and *Lactobacillus plantarum* strain MG-LAB302 were added to the wells at 10 µg/mL and were cultured. The culture was performed at 37°C under 5% CO₂. As a control, the RPMI medium not containing lactic acid bacteria was used.

### (ELISA assay)

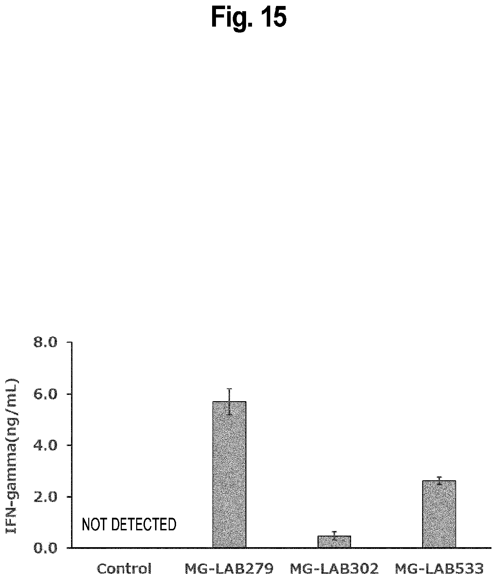

The mouse splenic cells were cultured for 20 hours, and 100 µL of the supernatant was collected from each well of the 96-well culture plate. The production level of each sample was quantitatively measured using an ELISA MAXTM Deluxe Set Mouse IFN-γ (BioLegend). Specifically, samples were prepared according to the attached protocol, and the absorbance of each sample was then measured at a wavelength of 450 nm. The production level of IFN-λ was quantitatively measured using a standard curve produced from the standard samples attached to the assay kit. The measurement results are shown in Figure 15. The measurement values are shown by averages of triplicate, and error bars indicate standard errors.

As shown in Figure 15, it was demonstrated that *Lactobacillus sakei* strain MG-LAB279 potently induces IFN-λ production. It was inferred that IL-12p70 induces differentiation of helper T precursor cells (Th0) to helper T cell type 1 (Th1), which accelerates IFN-γ production to enhance the activity of phagocytic cells, such as T cells and monocytes, and further activate NK cells.

### [Test Example 10]

### [Test for promoting production of IL-10 by human monocytic leukemia cell]

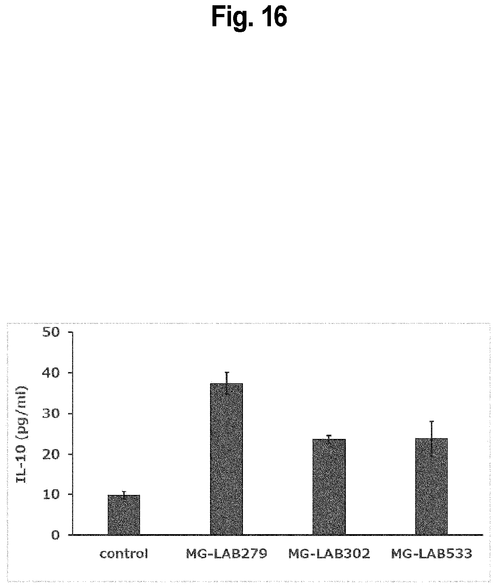

Human monocytic leukemia cell THP-1 purchased from JCRB Cell Bank was cultured in an RPMI 1640 (GIBCO) medium containing 10% fetal bovine serum (FBS, BIOWEST) and 100 units/mL of antibiotics (penicillin-streptomycin) (GIBCO) (hereinafter, RPMI 1640 medium) at 37°C under 5% CO₂ condition so as to give 1.0 × 10⁶ cells/mL. Subsequently, in order to differentiate the cells into macrophages, phorbol 12-myristate 13-acetate (PMA) was added thereto at a final concentration of 5 ng/mL, followed by culturing for 24 hours. Differentiation into macrophages was verified through a microscope, the culture supernatant was removed, washing was performed, fresh RPMI medium was then added thereto, and culture was performed for 24 hours. The cells were collected with 0.25 w/v% trypsin / 1 mmol/L EDTA·4Na solution (FUJIFILM Wako Pure Chemical Corporation) and were seeded in a 96-well plate (IWAKI) at 2.0 × 10⁴ cells/well. At 1 hour after the seeding, dry bacterial cell powders of *Lactobacillus sakei* strain MG-LAB279, *Pediococcus pentosaceus* strain MG-LAB533, and *Lactobacillus plantarum* strain MG-LAB302 were suspended in the RPMI 1640 medium and were added at a final concentration of 100 ug/mL each, followed by culturing. After culturing for 24 hours, the medium was collected, and the IL-10 concentration in the medium was measured using IL-10 Human Uncoated ELISA Kit (Invitrogen). The measurement results are shown in Figure 16. The measurement values are shown by averages of triplicate, and error bars indicate standard errors.

As shown in Figure 16, strain MG-LAB279 promoted the production of IL-10 by human monocytic leukemia cells. Accordingly, it was demonstrated that strain MG-LAB279 has a high activity of inducing anti-inflammatory cytokine production. This suggests a possibility of being effective for prevention and treatment of inflammatory disease including disease caused by virus infection.

### [Test Example 11]

### [Anti-inflammation test by dextran sulfate sodium using colitis model mouse]

### (Breeding of mouse)

The test was performed using 8-week old female C57BL/6J mice (Charles River Laboratories Japan, Inc.) after preliminary breeding for one week. The animals were bred in a breeding room that was maintained at controlled temperature: 20°C to 26°C, controlled humidity: 40% to 70% RH, and light and darkness for 12 hours each (lighting: 7:00 a.m. to 7:00 p.m.). A sterilized polycarbonate flat-bottom cage (213 mm × 324 mm × 131 mm) containing animal bedding (Pulsoft, Oriental Yeast Co., Ltd.) was used from the period of the preliminary breeding. In order to consider the breeding environment, the animal bedding was replaced by new one twice a week.

### (Grouping of mice)

The mice were divided into 3 groups: a group fed with a normal diet (hereinafter, referred to as "control group"); a group fed with a normal diet and subjected to inflammation induction by dextran sulfate sodium (DSS) (hereinafter, referred to as "DSS group"); and a group administered with the lactic acid bacterium *Lactobacillus sakei* strain MG-LAB279 and subjected to inflammation induction (hereinafter, referred to as "strain 279 administration group"), consisting of 4 mice in the control group and 8 mice in each of the other two groups..

### (Test food and administration method)

The test food in the control group and the DSS group was powder feed (MF, Oriental Yeast Co., Ltd.). The test food in the strain 279 administration group was the powder feed mixed with 0.5% of dead bacterial cells of the lactic acid bacterium *Lactobacillus sakei* strain MG-LAB279. After the grouping, the powder feed (MF) or the feed mixed with a test substance was placed in a feeder (MF-4S, SHIN FACTORY) and was freely ingested. The feed was supplied or replaced by new one twice a week. Administration of the test food was continued until the end of the test.

### (Inflammation induction and method for measuring body weight)

The 8-week old C57BL/6J mice in each group were freely fed with the feed until the end of the test. In the DSS group and the strain 279 administration group, colitis was induced by changing the drinking water to a 2.5% DSS aqueous solution on the 8th day of breeding and freely drinking it. The body weight of each mouse was measured on the day before the start (8th day) of DSS administration and the 11th, 13th, and 14th days. Figure 12 shows body weight change rates on the 11th, 13th, and 14th days compared to the body weight before the start of DSS ingestion.

### (Measurement of length of large intestine and colitis symptom score)

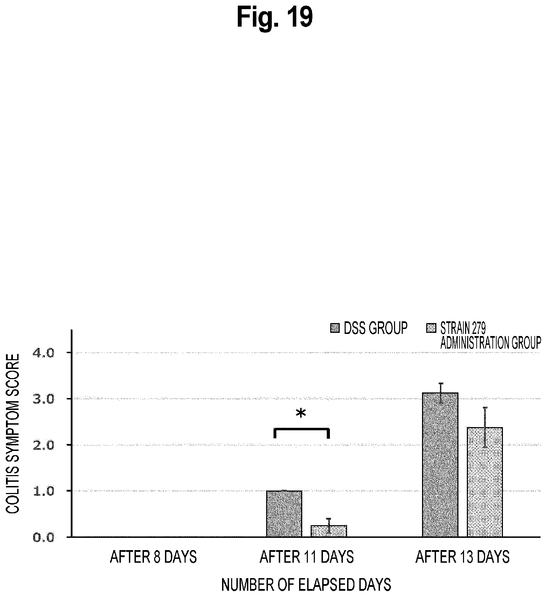

On the 14th day after start of the test, autopsy was performed under isoflurane anesthesia, and the length of the large intestine (from the distal end of the cecum to the anus) was measured. The colitis symptom score was measured on the day of the start (8th day) of inflammation induction and the 11th and 13th days. The colitis symptom scores shown in Figure 19 were calculated as total score of diarrhea and bloody stool symptoms of observed stool using the colitis symptom score sheet of Table 6. Figure 19 shows the colitis symptom scores on the day of the start (8th day) of inflammation induction and the 11th and 13th days over time. The score values are each represented by mean ± standard error. Verification was performed by two-group comparison (unpaired t-test) between the DSS group and the strain 279 administration group. The significance level was set at 5% ("*" in the Figure).

**[Table 6]**

| Score | Diarrhea | Bloody stool |
|---|---|---|
| 0 | Normal | Normal |
| 1 | Loose stool (stool having a high water content and having a shape) | Visible blood in the stool |
| 2 | Diarrheal stool (stool having a high water content and having no shape) | Blood adheres to the anus |
| 3 | Watery stool (liquid stool) | Continuous bleeding from the anus |

### (Result 1: Effect on body weight change rate)

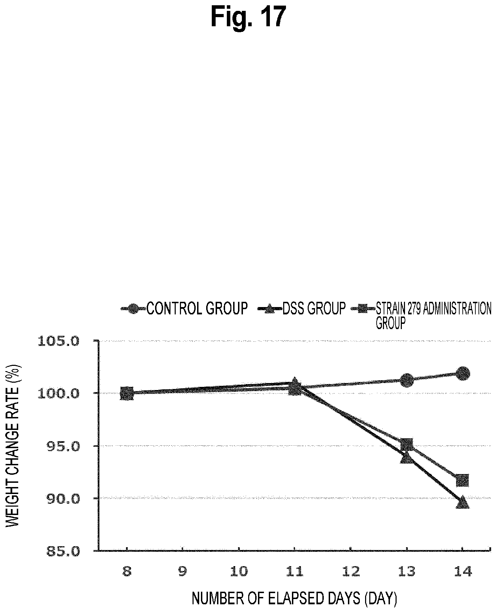

As shown in Figure 17, in contrast that the body weight increased in the control group, in the DSS group, the body weight decreased. On the other hand, also in the strain 279 administration group, a decrease in the body weight by DSS ingestion was observed, but the decrease was milder compared to the DSS group.

### (Result 2: Effect on length of large intestine and colitis symptom score)

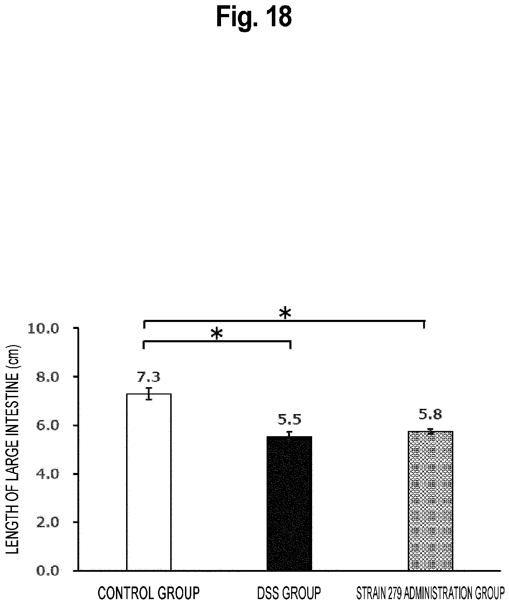

As shown in Figure 18, it was observed that the large intestine in the DSS group was shortened compared to the control group. Also in the strain 279 administration group, shortening of the large intestine by DSS ingestion was observed, but it was suppressed compared to the DSS group. As shown in Figure 19, no colitis symptoms appeared on the day of the start (8th day) of inflammation induction, but the colitis symptom score of the DSS group increased on the 11th and 13th days. In contrast, also in the strain 279 administration group, an increase in the colitis symptom score by DSS ingestion was observed, but it was less than that in the DSS group. This demonstrated that the lactic acid bacterium *Lactobacillus sakei* strain MG-LAB279 can delay and mitigate the onset of colitis symptoms induced by

### DSS.

### [Manufacturing example 1] (Culture of lactic acid bacterium)

The lactic acid bacterium *Lactobacillus sakei* strain MG-LAB279 was cultured in a commercially available medium. The cells were collected by centrifugation, and water was added thereto for resuspension, followed by centrifugation to obtain a concentrated bacterial cell solution. This was sterilized at 105°C or more for 30 minutes. The concentrated bacterial cell solution was then spray-dried to obtain a dead bacterial cell powder. This powder was mixed with dextrin (manufactured by San-ei Sucrochemical Co., Ltd., NSD300) to obtain a 10 mass% dead bacterial cell powder.

| | | |
|---|---|---|
| 0-1 | Form PCT/RO/134 | |
| 0-1-1 | The identification of this deposited microorganism or another biomaterial (PCT Rule Art. 13-2) was prepared by the method shown right. | JPO-PAS |
| | | i480 |
| 0-2 | International application number | |
| 0-3 | Document symbol of applicant or agent | M0179AOP0018 |
| | | |
| 1 | The identification below is associated with the microorganisms or biomaterials recited in the Detailed Description of the Invention Paragraph No. | 0018 |
| 1-1 | | |
| 1-3 | Identification of depository | |
| 1-3-1 | Title of depositary organization | NPMD (Independent administrative agency) National Institute of Technology and Evaluation, NITE Patent Organisms Depositary (NPMD) |
| 1-3-2 | Adress of depositary | Room 122, 2-5-8 Kazusakamatari, Kisarazu-shi, Chiba-ken, 292-0818, Japan |
| 1-3-3 | Deposit date | May 2, 2022 (02, 05, 2022) (02.05.2022) |
| 1-3-4 | Deposit No. | NPMD NITE BP-03645 |
| 1-5 | Designated countries to make this identification | All designated countries |
| 2 | The identification below is associated with the microorganisms or biomaterials recited in the Detailed Description of the Invention Paragraph No. | 0038 |
| 2-1 | | |
| 2-3 | Identification of depository | |
| 2-3-1 | Title of depositary organization | NPMD (Independent administrative agency) National Institute of Technology and Evaluation, NITE Patent Organisms Depositary (NPMD) |
| 2-3-2 | Adress of depositary | Room 122, 2-5-8 Kazusakamatari, Kisarazu-shi, Chiba-ken, 292-0818, Japan |
| 2-3-3 | Deposit date | May 2, 2022 (02, 05, 2022) (02.05.2022) |
| 2-3-4 | Deposit No. | NPMD NITE BP-03646 |
| 2-5 | Designated countries to make this identification | All designated countries |
| 3 | The identification below is associated with the microorganisms or biomaterials recited in the Detailed Description of the Invention Paragraph No. | 0038 |
| 3-1 | | |
| 3-3 | Identification of depository | |
| 3-3-1 | Title of depositary organization | NPMD (Independent administrative agency) National Institute of Technology and Evaluation, NITE Patent Organisms Depositary (NPMD) |
| 3-3-2 | Adress of depositary | Room 122, 2-5-8 Kazusakamatari, Kisarazu-shi, Chiba-ken, 292-0818, Japan |
| 3-3-3 | Deposit date | May 2, 2022 (02, 05, 2022) (02.05.2022) |
| 3-3-4 | Deposit No. | NPMD NITE BP-03647 |
| 3-5 | Designated countries to make this identification | All designated countries |

### Entry column for receiving office

| | | |
|---|---|---|
| 0-4 | This sheet was received with the international application (Yes/No) | √ |
| 0-4-1 | Authorized personnel | Yoko Yano |

### Entry column for International Bureau

| | | |
|---|---|---|
| 0-5 | The date this sheet was received by the international bureau | |
| 0-5-1 | Authorized personnel | |

## Claims

1. *Lactobacillus sakei* strain MG-LAB279 (accession number: NITE BP-03645).

2. A composition comprising the strain according to Claim 1 and/or a bacterial cell component thereof.

3. An immunostimulatory composition comprising the strain according to Claim 1 and/or a bacterial cell component thereof as an active ingredient.

4. The immunostimulatory composition according to Claim 3, wherein the immunostimulatory composition does not induce inflammation.

5. A virus infection preventing or treating composition comprising the strain according to Claim 1 and/or a bacterial cell component thereof as an active ingredient.

6. An NK cell-activating composition comprising the strain according to Claim 1 and/or a bacterial cell component thereof as an active ingredient.

7. An interferon λ production-promoting composition comprising the strain according to Claim 1 and/or a bacterial cell component thereof as an active ingredient.

8. An interleukin-12p70 production-promoting composition comprising the strain according to Claim 1 and/or a bacterial cell component thereof as an active ingredient.

9. A stress relief composition comprising the strain according to Claim 1 and/or a bacterial cell component thereof as an active ingredient.

10. An inflammation suppression composition comprising the strain according to Claim 1 and/or a bacterial cell component thereof as an active ingredient.

11. A skin-improving composition comprising the strain according to Claim 1 and/or a bacterial cell component thereof as an active ingredient.

12. Food or beverage comprising strain MG-LAB279 according to Claim 1 or the composition according to any one of Claims 2 to 11.

13. A pharmaceutical product comprising strain MG-LAB279 according to Claim 1 or the composition according to any one of Claims 2 to 11.
